# EUROPEAN PATENT APPLICATION

(11) **EP 2 044 941 A1**
(43) Date of publication of application: **08.04.2009**
(21) Application number: 08015966.8
(22) Date of filing: 31.08.2000
(51) Int. Cl.: A61K 31/565, A61K 31/585, A61P 15/18, A61K 9/20, A61K 9/28

(54) **Multiple-phase formulation of drospirenone and estradiol as a contraceptive**

(30) Priority: 31.08.1999 US 386274; 31.08.1999 EP 99202826
(62) Divisional of application: 05076943.9
(71) Applicant: Bayer Schering Pharma AG, 13353 Berlin (DE)
(72) Inventor: Heil, Wolfgang, 21435 Stelle (DE); Heithecker, Renate, 14163 Berlin (DE); Hilmann, Juergen, 13351 Berlin (DE); Lipp, Ralph, Zionsville, IN 46077 (US)
(74) Representative: Plougmann & Vingtoft A/S

(57) **Abstract**

The present invention relates to a multiple-phase pharmaceutical preparation consisting of a number of separately packaged and individually removable daily dosage units placed in a packaging unit and intended for oral administration for a period of at least 21 consecutive days, wherein said daily dosage unit comprises a natural estrogen, such as estradiol in an amount of from 0.5 mg to 4 mg, and 2 to 4 mg of drospirenone, and wherein at least 70% of said drospirenone is dissolved from said dosage unit within 30 minutes, as determined by USP XXIII Paddle Method II using water at 37°C as the dissolution media and 50 rpm as the stirring rate.

## Description

### FIELD OF THE INVENTION

The present invention relates to a multiple-phase pharmaceutical preparation comprising drospirenone and a natural estrogen.

### BACKGROUND OF THE INVENTION

Oral contraceptives containing a combination of a gestagen and an estrogen component have been used since the 1960's. The earliest contraceptive preparations consisted of 21 tablets containing the combination of active agents and 7 tablets containing no active agent, and the amount of each active agent was the same in each tablet (the so-called one-phase preparations). Subsequently, preparations were developed that consisted of tablets containing different amounts and ratios of the active agents over the cycle of administration (the so-called multiple-phase preparations).

Contraceptive reliability is mainly provided by the gestagen component. The daily dosage should be at least the minimum of what is needed for the gestagen in question to inhibit ovulation effectively. The estrogen component acts to increase the ovulation inhibitory effect of gestagen and to ensure cycle stability. Since the introduction of oral contraceptives, the daily dosage of gestagen has been reduced through the development of new and more efficient gestagens than were present in the earlier contraceptive preparations. It has also been possible to reduce the daily dosage of estrogen.

6β,7β;15β,16β-dimethylene-3-oxo-17α-pregn-4-ene-21,17-carbolactone (drospirenone) is known from DE 26 52 761 in which its use as a diuretic compound is disclosed.

In DE 30 22 337, the gestagen-like activity of the compound and its consequent utility as a contraceptive agent is described at dosage levels of 0.5-50 mg of drospirenone per day. It is also noted that the mechanism of action of the compound is very similar to that of the natural corpus luteum hormone progesterone, and that it does not give rise to increased blood pressure for which reason it may be administrated to women who have or are at risk of developing increased blood pressure. It is further described that drospirenone may be administered together with ethinylestradiol in an amount of 0.03-0.05 mg per day.

DE 30 51 166 discloses the use of the drospirenone for the treatment of gynaecological irregularities and for contraception at a dosage level of 0.5-50 mg per day.

EP 398 460 discloses the use of drospirenone for the treatment of androgen-induced disorders, aldosterone-induced disorders and hormonal irregularities as well as for contraception at dosage levels of 0.5-50 mg, preferably 1-10 mg per day. Ethinylestradiol may be co-administered at a level of 0.02-0.04 mg per day.

US 5,756,490 discloses pharmaceutical combination preparations comprising 23 or 24 dosage units containing a combination of a gestagen and an estrogen and 4-10 dosage units containing estrogen alone. Drospirenone is mentioned as a possible, but not preferred, gestagenic compound and ethinylestradiol is mentioned as a possible, but not preferred, estrogenic compound.

### SUMMARY OF THE INVENTION

In the course of research leading to the present invention, it has surprisingly been found that a hitherto undisclosed minimum dosage level of drospirenone is required for reliable contraceptive activity. Similarly, a preferred maximum dosage has been identified at which unpleasant side effects, in particular excessive diuresis, may substantially be avoided.

Accordingly, in a first aspect, the present invention relates to a pharmaceutical composition comprising, as a first active agent, 6β,7β;15β,16β-dimethylene-3-oxo-17α-pregn-4-ene-21,17-carbolactone (drospirenone) in an amount corresponding to a daily dosage, on administration of the composition, of from about 2 mg to about 4 mg, and, as a second active agent, 17α-ethinylestradiol (ethinylestradiol) in an amount corresponding to a daily dosage of from about 0.01 mg to about 0.05 mg, together with one or more pharmaceutically acceptable carriers or excipients.

Apart form the active substances themselves, it is envisaged that an ester or prodrug of drospirenone may be employed in the present composition, e.g. an oxyiminopregnane carbolactone as disclosed in WO 98/24801. Likewise, it is envisaged that esters or ethers of ethinylestradiol may be included in the composition.

In a further aspect, the invention relates to a method of inhibiting ovulation in a mammal, in particular a human, comprising administering, to said mammal, drospirenone in an amount in the range of from about 2 mg to about 4 mg of per day, together with ethinylestradiol in an amount of from about 0.01 mg to about 0.05 mg per day, said amounts being effective to inhibit ovulation in said mammal.

In a still further aspect, the invention relates to the use of drospirenone combined with ethinylestradiol for preparing a pharmaceutical preparation for the inhibition of ovulation in a mammal, in particular a human, the composition comprising an amount of drospirenone corresponding to a daily dosage, on administration of the composition, of from about 2 mg to about 4 mg, and comprising an amount of ethinylestradiol corresponding to a daily dosage, on administration of the composition, of from about 0.01 to about 0.05 mg.

### BRIEF DESCRIPTION OF THE DRAWINGS

The invention is further described with reference to the drawings in which
Fig. 1 is a graph showing the in vitro dissolution rate of drospirenone from tablet cores, V1-V7 being batches containing micronized drospirenone, and V8 being a batch containing macrocrystalline drospirenone;
Fig. 2 is a graph showing the in vitro dissolution rate of drospirenone from tablet cores, different lines representing different test batches;
Fig. 3 is a graph showing the in vitro dissolution rate of drospirenone from film-coated tablets, different lines representing different test batches;
Fig. 4 is a graph showing the in vitro dissolution rate of ethinylestradiol from tablet cores, different lines representing different test batches; and
Fig. 5 is a graph showing the in vitro dissolution rate of ethinylestradiol from film-coated tablets, different lines representing different test batches.

### DETAILED DISCLOSURE OF THE INVENTION

Drospirenone, which may be prepared substantially as described in, e.g., US 4,129,564 or WO 98/06738. is a sparingly soluble substance in water and aqueous buffers at various pH values. Furthermore, drospirenone is rearranged to an inactive isomer under acid conditions and hydrolysed under alkaline conditions. To ensure good bioavailability of the compound, it is therefore advantageously provided in a form that promotes rapid dissolution thereof.

It has surprisingly been found that when drospirenone is provided in micronized form (so that particles of the active substance have a surface area of more than 10,000 cm²/g, and the following particle size distribution as determined under the microscope: not more than 2 particles in a given batch with a diameter of more than 30 µm, and preferably ≤ 20 particles with a diameter of ≥ 10 µm and 5 30 µm) in a pharmaceutical composition, rapid dissolution of the active compound from the composition occurs in vitro ("rapid dissolution" is defined as the dissolution of at least 70% over about 30 minutes, in particular at least 80% over about 20 minutes, of drospirenone from a tablet preparation containing 3 mg of drospirenone in 900 ml of water at 37°C determined by the USP XXIII Paddle Method using a USP dissolution test apparatus 2 at 50 rpm). Instead of providing the drospirenone in micronized form, it is possible to dissolve it in a suitable solvent, e.g. methanol or ethyl acetate, and spray it onto the surface of inert carrier particles followed by incorporation of the particles containing drospirenone on their surface in the composition.

Without wishing to be limited to any particular theory, it appears that the in vitro dissolution rate of drospirenone is connected to the dissolution rate in vivo resulting in rapid absorption of drospirenone in vivo on oral administration of the compound. This is an advantage because isomerization of the compound in the gastric environment and/or hydrolysis in the intestine is substantially reduced, leading to a high bioavailability of the compound.

With respect to ethinylestradiol which is also a sparingly soluble substance, though less sensitive to degradation than drospirenone under conditions prevailing in the gastrointestinal tract, it is also an advantage to provide it in micronized form or sprayed from a solution, e.g. in ethanol, onto the surface of inert carrier particles. This has the added advantage of facilitating a more homogenous distribution of the ethinylestradiol throughout the composition which might otherwise be difficult to obtain because ethinylestradiol is incorporated in extremely small amounts. When ethinylestradiol is provided in micronized form, it preferably has the following particle size distribution as determined under the microscope: 100% of the particles have a diameter of ≤ 15.0 µm, 99% of the particles have a diameter of ≤ 12.5µm, 95% of the particles have a diameter of ≤ 10.0 µm, and 50% of the particles have a diameter of ≤ 3.0 µm. Furthermore, no particle is larger than 20 µm, and ≤ 10 particles have a diameter of ≥ 15 µm and ≤ 20 µm.

To obtain a more rapid rate of dissolution, it is preferred to include carriers or excipients which act to promote dissolution of both active substances. Examples of such carriers and excipients include substances that are readily soluble in water such as cellulose derivatives, carboxymethylcellulose, hydroxypropyl cellulose, hydroxypropylmethyl cellulose, gelled starch, gelatin or polyvinylpyrrolidone. In particular, it appears as though polyvinylpyrrolidone might be particularly helpful to promote dissolution.

The composition of the invention preferably comprises drospirenone in an amount corresponding to a daily dosage of from about 2.5 mg to about 3.5 mg, in particular about 3 mg. The amount of ethinylestradiol preferably corresponds to a daily dosage of from about 0.015 mg to about 0.04 mg, in particular from about 0.015 mg to about 0.03 mg.. More particularly, the present composition comprises an amount of drospirenone corresponding to a daily dosage of from about 3.0 to about 3.5 mg and ethinylestradiol in an amount corresponding to from about 0.015 to about 0.03 mg.

Apart from its ability to inhibit ovulation, the composition of the invention has been found to possess pronounced anti-androgenic properties and may therefore be used in the prevention or treatment of androgen-induced disorders, in particular acne. Such use may be independent from or concomitant with the use as a contraceptive disclosed above. Furthermore, since drospirenone is an aldosterone antagonist, it has diuretic properties and is therefore suitable for counteracting the water-retentive properties of ethinylestradiol.

In a particular embodiment, the invention relates to a pharmaceutical preparation consisting of a number of separately packaged and individually removable daily dosage units placed in a packaging unit and intended for oral administration for a period of at least 21 consecutive days, wherein each of said daily dosage units comprises a combination of drospirenone in an amount of from about 2 mg to about 4 mg and ethinylestradiol in an amount from about 0.01 to about 0.05 mg.

In one embodiment, the preparation further comprises 7 or less said daily dosage units containing no active agent. Alternatively, it is possible to include, in the dosage regimen, a period of 7 days or less during which no dosage units are ingested. For compliance reasons, however, it may be preferred to include an appropriate number of blanks in the preparation, in which case the total number of daily dosage units in the preparation is at least 28. The inclusion of blanks, or the pill-free days, will then trigger withdrawal bleeding.

The preparation may be a one-phase composition, i.e. a preparation wherein the amounts of either active agent remains constant for the entire at least 21-day period, or the amounts of either or both active agents may be varied over the at least 21-day period to generate a multiple-phase preparation, e.g. a two- or three-phase preparation, substantially as disclosed in, e.g., EP 148 724. In case of multiple-phase preparation, it may be possible to include a natural estrogen such as estradiol, e.g. in an amount of from about 0.5 mg to about 4 mg per day, instead of ethinylestradiol.

In suitable embodiments of the present preparation, the number of daily dosage units comprising the combination of drospirenone and ethinylestradiol may be 21, 22, 23 or 24, and the number of daily dosage units containing no active agent may then be 7, 6, 5 or 4, as the case may be. In a further embodiment of the present preparation, the number of daily dosage units comprising the combination of drospirenone and ethinylestradiol is 28. or a multiple of 28 such as 2-4, in particular 2 or 3, times 28.

In an alternative embodiment, the invention relates to a contraceptive preparation consisting of a number of separately packaged and individually removable daily dosage units placed in a packaging unit and intended for oral administration for a period of at least 28 consecutive days, wherein at least 21 of said daily dosage units comprises a combination of drospirenone in an amount of from about 2 mg to about 4 mg and ethinylestradiol in an amount from about 0.01 to about 0.05 mg, and wherein 7 or less of said daily dosage units contain ethinylestradiol alone in an amount from about 0.01 to about 0.05 mg.

By including an appropriate number of dosage units comprising ethinylestradiol alone, high contraceptive reliability, low follicular development and satisfactory cycle control with little or no intermenstrual bleeding may be obtained.

In this case, too, the preparation may be one in which the amounts of either active agent remains constant for the entire at least 21-day period (i.e. a two-phase preparation), or the amounts of either or both active agents may be varied over the at least 21-day period to generate a multiple-phase preparation, e.g. a three- or four-phase preparation, substantially as disclosed in, e.g., EP 148 724. In case of multiple-phase preparation, it may be possible to include a natural estrogen such as estradiol, e.g. in an amount of from about 0.5 mg to about 4 mg per day, instead of ethinylestradiol.

In suitable embodiments of the present preparation, the number of daily dosage units comprising the combination of drospirenone and ethinylestradiol may be 21, 22, 23 or 24, and the number of daily dosage units containing ethinylestradiol alone may then be 7, 6, 5 or 4, as the case may be.

In one embodiment of the present method of inhibiting ovulation, the method comprises administering, to said mammal, on each day of at least 21 consecutive days, a daily dosage unit comprising a combination of drospirenone in an amount of from about 2 mg to about 4 mg and ethinylestradiol in an amount from about 0.01 to about 0.05 mg, followed by administering, on each day of 7 or less consecutive days, a daily dosage unit containing no active agent, or alternatively, administering no dosage units for 7 days or less.

In suitable embodiments of this method, the daily dosage units comprising the combination of drospirenone and ethinylestradiol may be administered for 21, 22, 23 or 24 consecutive days, and the daily dosage units containing no active agent may then be administered for 7, 6, 5 or 4 consecutive days, as appropriate. Furthermore, the daily dosage units comprising the combination of drospirenone and ethinylestradiol may be administered for 28 consecutive days. In a variant of this embodiment, the daily dosage units comprising the combination of drospirenone and ethinylestradiol are administered for 2-4, preferably 2 or 3, times 28 consecutive days, followed by administration of the daily dosage units comprising the combination of drospirenone and ethinylestradiol for 21, 22, 23 or 24 consecutive days and subsequently administration of the daily dosage units containing no active agent, or administration of no daily dosage units, for 7, 6, 5 or 4 consecutive days.

Alternatively, the present method comprises administering, on each day of at least 21 consecutive days, a daily dosage unit comprising a combination of drospirenone in an amount of from about 2 mg to about 4 mg and ethinylestradiol in an amount from about 0.01 to about 0.05 mg, followed by administering, on each day of 7 or less consecutive days, a daily dosage unit containing ethinylestradiol alone in an amount of from about 0.01 mg to about 0.05 mg.

In this alternative method, the daily dosage units comprising the combination of drospirenone and ethinylestradiol may suitably be administered for 21, 22, 23 or 24 consecutive days, and wherein the daily dosage units comprising ethinylestradiol alone may then be administered for 7, 6, 5 or 4 consecutive days, as appropriate. In a further embodiment of the method, the daily dosage units comprising the combination of drospirenone and ethinylestradiol are administered for 2-4, preferably 2 or 3, times 28 consecutive days, followed by administration of the daily dosage units comprising the combination of drospirenone and ethinylestradiol for 21 consecutive days and subsequently administration of the daily dosage units comprising ethinylestradiol alone for 7 consecutive days.

For use according to the invention, the pharmaceutical preparation may suitably be in the form of a number of separately packaged and individually removable daily dosage units placed in a packaging unit and intended for oral administration for a period of at least 21 consecutive days, wherein each of said daily dosage units each comprises a combination of drospirenone in an amount of from about 2 mg to about 4 mg and ethinylestradiol in an amount from about 0.01 to about 0.05 mg.

As indicated above, the preparation may further comprise 7 or less daily dosage units containing no active agent (or may contain 7 or less empty "places", e.g. in the form of empty blisters in a blister pack, marking the days on which no daily dosage units are administered).

Alternatively, the pharmaceutical preparation may be in the form of a number of separately packaged and individually removable daily dosage units placed in a packaging unit and intended for oral administration for a period of at least 28 consecutive days, wherein at least 21 of said daily dosage units each comprises a combination of drospirenone in an amount of from about 2 mg to about 4 mg and ethinylestradiol in an amount of from about 0.01 to about 0.05 mg, said packaging unit further comprising 7 or less daily dosage units comprising ethinylestradiol alone in an amount of from about 0.01 mg to about 0.05 mg.

The composition of the invention may be formulated in any manner known in the pharmaceutical art. In particular, as indicated above, the composition may be formulated by a method comprising providing drospirenone and, if desired, ethinylestradiol in micronized form in said unit dosage form, or sprayed from a solution onto particles of an inert carrier in admixture with one or more pharmaceutically acceptable excipients that promote dissolution of the drospirenone and ethinylestradiol so as to promote rapid dissolution of drospirenone and preferably ethinylestradiol on oral administration. Examples of suitable excipients include fillers, e.g. sugars such as lactose, glucose or sucrose, sugar alcohols such as mannitol, sorbitol or xylitol, starch such as wheat, corn or potato starch, modified starch or sodium starch glycolate, lubricants such as talc, magnesium stearate, calcium stearate, colloidal silica or stearic acid, and binders such as polyvinylpyrrolidone, cellulose derivatives, carboxymethyl cellulose, hydroxypropyl cellulose, hydroxypropylmethyl cellulose, methyl cellulose or gelatin, for making oral dosage forms such as tablets, pills or capsules.

Tablets may conveniently be coated with a suitable film-forming agent, e.g. hydroxypropylmethyl cellulose, hydroxypropyl cellulose or ethyl cellulose, to which a suitable excipient may optionally be added, e.g. a softener such as glycerol, propylene glycol, diethylphthalate or glycerol triacetate, filler such as sucrose, sorbitol, xylitol, glucose or lactose, a colorant such as titanium hydroxide, etc.

The present composition may also be formulated in liquid form, e.g. as a solution, suspension or emulsion, together with conventional diluents or excipients in a manner known per se in the pharmaceutical art.

A packaging unit comprising the daily dosage units described above may be prepared in a manner analogous to that of making other oral contraceptives. This may for instance be a conventional blister pack or any other form known for this purpose, for instance a pack comprising the appropriate number of dosage units (in this case at least 21, or for particular applications, 28 or a multiple of 28) in a sealed blister pack with a cardboard, paperboard, foil or plastic backing and enclosed in a suitable cover. Each blister container may conveniently be numbered or otherwise marked, e.g. starting with the first of the at least 21 dosage units that contain the combination of drospirenone and ethinylestradiol, optionally followed by 7 or less empty blisters or by the 7 or less dosage units that contain no active agent or that only contain ethinylestradiol (although the numbering may also start with the first of the 7 or less dosage units that only contain ethinylestradiol).

It is also envisaged that the present composition may be in the form of a parenteral formulation such as a subcutaneous implant or transdermal formulation. For making implants, the active agents may suitably be formulated together with one or more polymers that are gradually eroded or degraded when in use, e.g. silicone polymers; ethylene vinylacetate, polyethylene or polypropylene.

Where transdermal formulations are concerned, they may be prepared in the form of matrices or membranes or as fluid or viscous formulations in oil or hydrogels. For transdermal patches, an adhesive which is compatible with the skin should be included, such as polyacrylate, a silicone adhesive or polyisobutylene, as well as a foil made of, e.g. polyethylene, polypropylene, ethylene vinylacetate, polyvinylchloride, polyvinylidene chloride or polyester, and a removable protective foil made from, e.g., polyester or paper coated with silicone or a fluoropolymer. For the preparation of transdermal solutions or gels, water or organic solvents or mixtures thereof may be used. Transdermal gels may furthermore contain one or more suitable gelling agents or thickeners such as silicone, tragacanth, starch or starch derivatives, cellulose or cellulose derivatives or polacrylic acids or derivatives thereof. Transdermal formulations may also suitably contain one or more substances that enhance absorption though the skin, such as bile salts or derivatives thereof and/or phospholipids. Suitable transdermal formulations may, for instance, be made in a manner analogous to that described in WO 94/04157 for 3-ketodesogestrel. Alternatively, transdermal formulations may be prepared according to a method disclosed in, e.g., BW Barry, "Dermatological Formulations, Percutaneous Absorption", Marcel Dekker Inc., New York - Basel, 1983, or YW Chien, "Transdermal Controlled Systemic Medications", Marcel Dekker Inc., New York - Basel, 1987.

The present invention is further described in the following examples which are not in any way intended to limit the scope of the invention as claimed.

### EXPERIMENTAL

### Example 1

### Preparation of tablets containing drospirenone and ethinylestradiol

**Tablet cores of the following composition**

| | |
|---|---|
| micronized drospirenone | 3.00 mg |
| micronized ethinylestradiol | 0.03 mg |
| lactose monohydrate | 48.17 mg |
| corn starch | 14.40 mg |
| modified starch | 9.60 mg |
| polyvinylpyrrolidone 25,000 | 4.00 mg |
| magnesium stearate | 0.80 mg |

were prepared by charging a fluidized bed granulator with 31.68 kg corn starch, 21.12 kg modified starch, 6.60 micronized drospirenone, 0.066 kg micronized ethinylestradiol and 105.974 kg lactose monohydrate and activating the fluidized bed. An aqueous solution of 8.80 kg polyvinylpyrrolidone 25,000 in 46.20 kg purified water was sprayed continuously onto the fluidized bed while drying by heating the air stream of the fluidized bed. At the end of the process 1.76 kg magnesium stearate was sucked into the granulator and mixed with the granules by maintaining the fluidized bed. The resulting granulate was pressed into tablet cores by compression using a rotary tablet press.

2.22464 kg of hydroxypropylmethylcellulose and 0.44528 macrogol 6000 were dissolved in 14.67 kg purified water. 0.44528 kg talc, 1.22430 kg titanium dioxide and 0.06050 kg ferric oxide pigment were suspended in 10.26 kg purified water with stirring and homogenized. The solution and suspension were combined and used to coat the tablet cores by continuous application of the coating suspension in a coater.

### Example 2

### Dissolution of drospirenone from tablets

The rate of dissolution of drospirenone from the tablets prepared in Example 1 was determined by the USP XXIII Paddle Method using a USP Dissolution Test Apparatus 2 including 6 covered glass vessels and 6 paddles. Tablets were placed in 900 ml water at a temperature of 37°C (± 0.5°C) and stirred at 50 rpm.

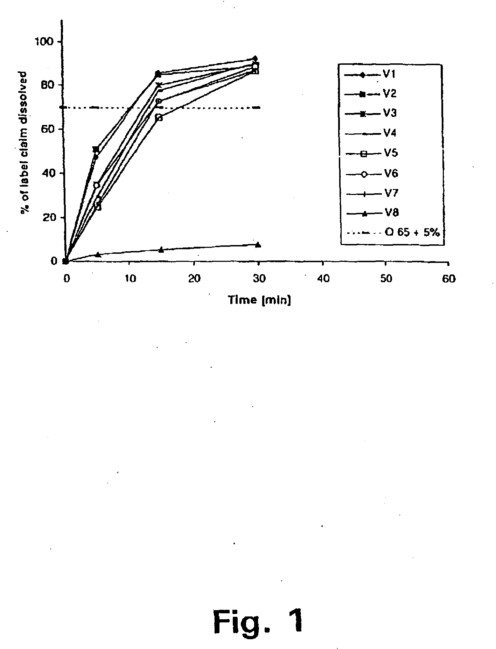

The results appear from Figs. 1, 2 and 4. From Fig. 1, it appears that the batch numbered V8 containing macrocrystalline drospirenone (but otherwise identical to the tablets prepared in Example 1) exhibited an extremely slow dissolution rate of drospirenone, whereas all batches containing micronized drospirenone exhibited a dissolution rate of more than 70% within 30 minutes.

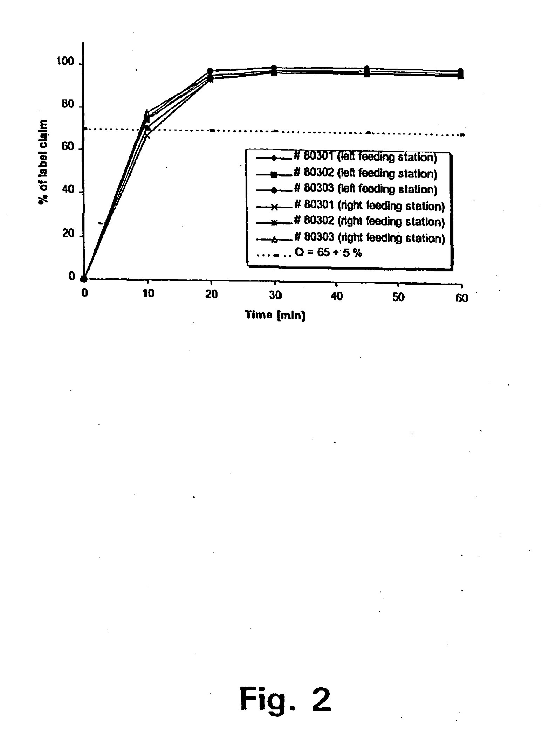

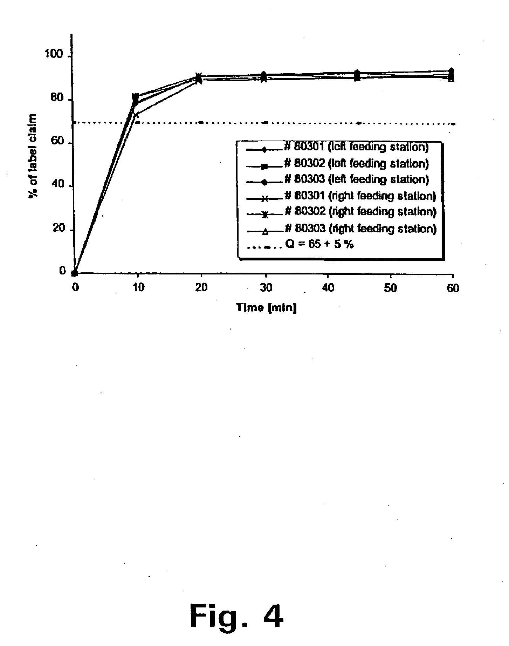

Fig. 2 and Fig. 4 shows the results of dissolution of drospirenone from tablet cores and film-coated tablets, respectively. In both cases more than 70% of the active agent is dissolved within 30 minutes. Thus, the film coating did not significantly influence the rate of dissolution.

### Example 3

### Dissolution rate of ethinylestradiol from tablets in vitro

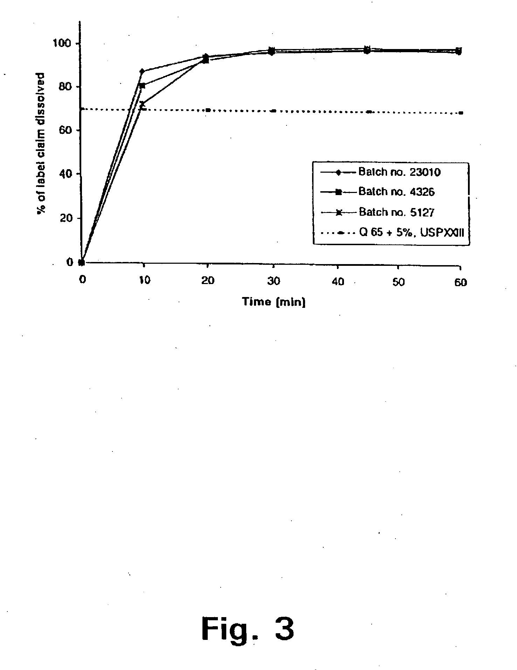

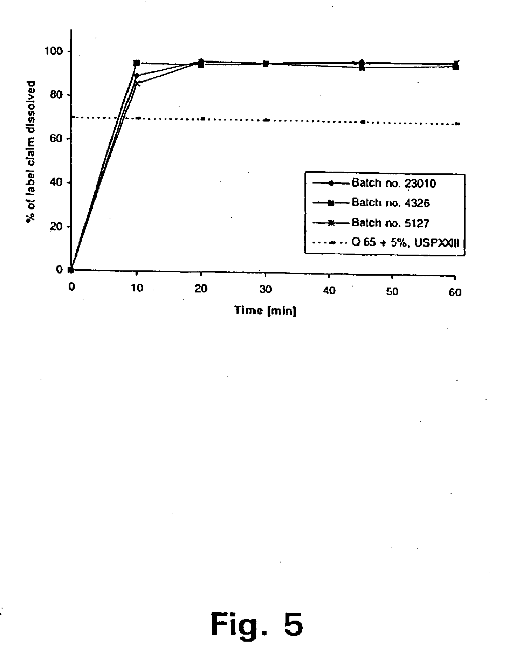

The rate of dissolution of ethinylestradiol from tablets prepared as described in Example 1 was determined according to the USP Paddle Method as described in Example 2 for drospirenone. The results appear from Figs. 3 and 5 showing the dissolution rates from tablet cores and film-coated tablets, respectively. In both cases, more than 70% of the active agent was dissolved within 30 minutes. Thus, the film coating did not significantly influence the rate of dissolution.

### Example 4

### Bioavailability of drospirenone and ethinylestradiol from tablets containing 3 mg of drospirenone and 0.03 mg ethinylestradiol

42 healthy women between 18 and 35 years of age were included in an open-label crossover study after their informed consent had been obtained. The aim of the study was to investigate the relative bioavailability of drospirenone and ethinylestradiol from a tablet formulation containing 3 mg drospirenone and 0.03 mg ethinylestradiol with reference to an oral suspension containing 6 mg of drospirenone and 0.06 mg ethinylestradiol per vial.

The bioavailability was determined using serum concentrations of each active agent as parameters. Compared to the oral suspension, the relative bioavailability of drospirenone and ethinylestradiol from the tablets is 107% and 117%, respectively. It was therefore concluded that both drospirenone and ethinylestradiol are completely released from the tablets in vivo.

The absolute bioavailability of drospirenone was determined in two studies to be 76%±13% after oral administration of 2 mg drospirenone to 8 young healthy women and 85%±24% after oral administration of a microcrystalline suspension containing 3.13 mg drospirenone to 6 postmenopausal women.

The oral bioavaliability of ethinylestradiol was determined in several studies, and mean values of from 36% to 59% were reported in the literature, indicating a first-pass effect.

### Example 5

### Contraceptive efficacy of formulations containing drospirenone and ethinylestradiol

An open-label, randomized trial with 52 female volunteers aged 20-35 years whose informed consent had been obtained included 1 pre-treatment cycle, 3 treatment cycles with two different tablet containing 2 mg and 3 mg drospirenone, respectively, but otherwise corresponding to the tablets prepared in Example 1, and a follow-up phase. A wash-out phase of 1 month preceded the treatment.

At defined time points, selected central and peripheral parameters were investigated: LH, FSH, 17β-estradiol, progesterone, cervical score, "spinnbarkeit", fern phenomenon. Ovarian function was checked by ultrasound. In addition, SHBG, CBG, prolactine, total testosterone, androstenedione, DHEA-S and selected metabolic parameters (serum glucose, triglycerides, cholesterol, HDL, LDL) were examined. Blood pressure, heart rate, body weight and cycle control were documented.

The results of the study showed that both LH and FSH were dearly suppressed with both trial preparations. Accordingly, the secretion of estradiol and progesterone were greatly reduced over all three treatment cycles with the exception of 3 volunteers receiving the 2 mg drospirenone preparation. This result was, in principle, confirmed by the accompanying ultrasound examinations. Follicular ripening occurred in several cases with both trial preparations. Although three ovulations were diagnosed with the preparation containing 2 mg drospirenone (one of which was described as "equivocal" and the other as a "tablet-taking error"), no differences were demonstrable statistically (p > 0.05) between the two trial preparations as regards the hormones LH, FSH, estradiol and progesterone, and the parameter "ovulation during the treatment cydes". In keeping with the hormones, cervical function was greatly limited and the "spinnbarkeit" and crystallisability of the cervical mucus was greatly reduced with both trial preparations. Prolactin increased minimally and SHBG and CBG distinctly with both preparations. Triglycerides and HDL levels increased with both trial preparations, while LDL levels decreased. Total cholesterol was largely unchanged in both treatment groups. Oral glucose tolerance remained virtually unchanged or was slightly decreased. Testosterone, androstenedione and DHEA-S decreased minimally.

The subjective and objective tolerance was good with both treatments. This was also the case for cycle control with the exception of the first cycle with 2 mg drospirenone. Blood pressure, heart rate and body weight remained constant in the majority of cases or showed a slight tendency to decrease.

After three months' treatment, it was concluded:
The two trial preparations were equally good as regards the subjective and objective tolerance.

No negative metabolic effects were observed with either preparation. HDL was influenced positively in the sense of an increase.

The results confirmed the results of earlier studies that the 2 mg drospirenone preparation was in the threshold region of ovulation inhibition; whereas the 3 mg drospirenone preparation had a demonstrable ovulation-inhibiting effect in all cases examined.

## Claims

1. A multiple-phase pharmaceutical preparation consisting of a number of separately packaged and individually removable daily dosage units placed in a packaging unit and intended for oral administration for a period of at least 21 consecutive days, wherein said daily dosage unit comprises a natural estrogen and 2 to 4 mg of drospirenone, and wherein at least 70% of said drospirenone is dissolved from said dosage unit within 30 minutes, as determined by USP XXIII Paddle Method II using water at 37°C as the dissolution media and 50 rpm as the stirring rate.

2. The multiple-phase pharmaceutical preparation according to claim 1, wherein said drospirenone is in micronized form.

3. The multiple-phase pharmaceutical preparation according to claim 1, wherein said drospirenone has a surface area of more than 10,000 cm²/g.

4. The multiple-phase pharmaceutical preparation according to claim 1, wherein said drospirenone is sprayed from a solution of drospirenone onto particles of an inert carrier.

5. The multiple-phase pharmaceutical preparation according to any of the preceding claims, wherein said natural estrogen is estradiol.

6. The multiple-phase pharmaceutical preparation according to claim 5, wherein said dosage unit comprises 0.5 to 4 mg of estradiol.

7. The multiple-phase pharmaceutical preparation according to any of the preceding claims, wherein said dosage unit comprises 2.5 to 3.5 mg of drospirenone.

8. The multiple-phase pharmaceutical preparation according to claim 7, wherein said dosage unit comprises about 3 mg of drospirenone.
